(19) Europäisches Patentamt
European Patent Office
Office européen des brevets

(11) Veröffentlichungsnummer: **0 222 163**
**B1**

(12) # EUROPÄISCHE PATENTSCHRIFT

(45) Veröffentlichungstag der Patentschrift: **19.07.89**

(51) Int. Cl.⁴: **C07D 207/273**, C07D 207/267, C07D 207/28

(21) Anmeldenummer: **86113938.4**

(22) Anmeldetag: **08.10.86**

(54) **Verfahren zur Herstellung von gamma-Butyrolactamen.**

(30) Priorität: **18.10.85 DE 3537074**
**25.09.86 DE 3632589**

(43) Veröffentlichungstag der Anmeldung: **20.05.87 Patentblatt 87/21**

(45) Bekanntmachung des Hinweises auf die Patenterteilung: **19.07.89 Patentblatt 89/29**

(84) Benannte Vertragsstaaten: **AT BE CH DE ES FR GB IT LI LU NL SE**

(56) Entgegenhaltungen:
**AT-B- 374 457**
**AT-B- 377 982**

**PLANTA MEDICA, Band 32, 1977, Stuttgart, I. MESTER et al. "Inhaltsstoffe aus Clausena anisata (Willd.) Oliv. (Rutaceae) I. Cumarine aus der Wurzelrinde", pp. 81-85**
**PHYTOCHEMISTRY, Band 17, 1978, Oxford, New York, Frankfurt, Sydney, DHAN RAKASH et al. "Coumarins from Clausena indica", pp. 1194, 1195**

(73) Patentinhaber: **BAYER AG, D-5090 Leverkusen 1 Bayerwerk(DE)**
Patentinhaber: **CHINESE ACADEMY OF MEDICAL SCIENCES, Institute of Materia Medica 1 Xian Nong Tan Street, Beijing(CN)**

(72) Erfinder: **Hartwig, Wolfgang, Dr., Pahlkestrasse 3, D-5600 Wuppertal 1(DE)**

(74) Vertreter: **Dänner, Klaus, Dr. et al, c/o Bayer AG Konzernverwaltung RP Patentabteilung, D-5090 Leverkusen 1 Bayerwerk(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von γ-Butyrolactamen, die wertvolle antiamnestische Wirkung besitzen.

Es ist bekannt, daß Rutaceae Clausena anicata in bestimmten Teilen Afrikas als Volksmedizin verwendet wird [I. Mester et al, Planta Medica 32, 81 (1977)]. Es ist ebenfalls bekannt, daß der rohe Extrakt von Clausena indica Oliv, cardiovaskuläre Aktivität besitzt und daß zwei aus Clausena pentaphylla (Roxb.) dünnschichtchromatographisch isolierte Cumarinderivate, Clausmarin A und B, spasmolytische Aktivität besitzen [Dhan Prakash et al, Phyochem. 17, 1194 (1978); Aboo Shoeb et al, J. Chem. Soc., Chem. Commun. 1978, 281]. Außerdem gilt der wäßrige Extrakt aus Blättern von Clausena Lansium (lour) Skeels in der chinesischen Volksmedizin als wirksames Leberschutzmittel und wird gegen akute und chronische Virushepatitis eingesetzt. Aus diesem Extrakt konnte als einer der Hauptbestandteile (±)3(S*), 4(R*), 7(S*)-3-Hydroxy-5-α-hydroxybenzyl-1-methyl-4-phenylpyrrolidin-2-on (Clausenamid) isoliert werden.

Clausenamid sowie die aus Clausenamid hergestellten Derivate zeigen im Tierversuch antiamnestische sowie vor cerebraler Hypoxie schützende Wirkung. Da für die weiteren pharmakologischen Studien größere Substanzmengen benötigt werden, andererseits durch das aufwendige Extraktionsverfahren nur 1,5 g Ausgangsverbindung aus 4 kg getrockneten Blättern erhältlich sind, war es notwendig, ein Verfahren zur chemischen Synthese zur Verfügung zu stellen.

Die vorliegende Erfindung betrifft ein Verfahren zur Herstellung von C(3)-C(4)-trans konfigurierten γ-Butyrolactamen der allgemeinen Formel (I),

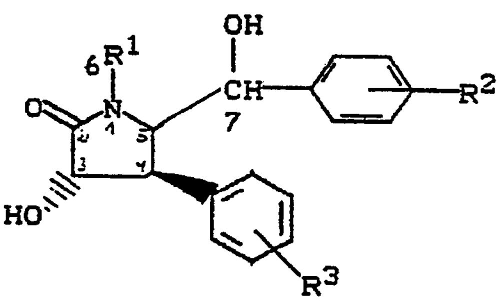

(I)

in welcher

$R^1$ für Wasserstoff, Alkyl, Aryl oder Aralkyl mit jeweils bis zu 10 Kohlenstoffatomen steht,

und

$R^2$ und $R^3$ gleich oder verschieden sind und für Wasserstoff, Alkyl, Aryl, Aralkyl, Alkoxy, Aryloxy oder Aralkoxy mit jeweils bis zu 10 Kohlenstoffatomen, Acyl mit bis zu 18 Kohlenstoffatomen, Trifluormethyl, Trifluormethoxy, Nitro, Hydroxy, Halogen, Amino, Carboxy, Sulfo, Dialkylamino mit bis zu 4 Kohlenstoffatomen in den Alkylgruppen oder Acylamino mit bis zu 18 Kohlenstoffatomen steht, mit der Ausnahme, daß $R^1$ nicht Methyl bedeutet, wenn $R^2$ und $R^3$ Wasserstoff bedeuten, in Form ihrer Isomeren, Isomerengemische, Racemate oder optischen Antipoden dadurch gekennzeichnet, daß man Verbindungen der allgemeinen Formel (II)

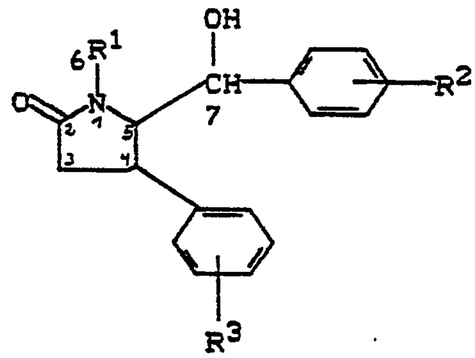

(II)

in welcher

$R^1$, $R^2$ und $R^3$ die oben angegebene Bedeutung haben, in inerten organischen Lösungsmitteln in Anwesenheit einer Base, gegebenenfalls in Gegenwart eines Hilfsstoffes oxidiert.

Es ist überraschend, daß mit Hilfe des erfindungsgemäßen Verfahrens ausschließlich das C(3)-C(4)-trans-konfigurierte Hydroxylierungsprodukt (I) in guter Ausbeute ensteht. Das erfindungsgemäße Verfahren hat den Vorteil, daß in kurzer Zeit und mit wenig Aufwand größere Substanzmengen zur Verfügung gestellt werden können. Außerdem ist es möglich, mit der Wahl der Ausgangsverbindung (II) die

Stereochemie der Endprodukte zu bestimmen, d.h. gezielt einzelne Stereoisomere herzustellen.

In der vorstehenden Definition enthalten die "Alkyl" – und "Alkoxy"-Gruppen vorzugsweise bis zu 6 Kohlenstoffatome, "Aryl", "Aralkyl", "Aryloxyl" und "Aralkoxy" bedeuten vorzugsweise Phenyl, Benzyl, Phenoxy bzw. Benzyloxy und "Acyl" -Gruppen enthalten vorzugsweise bis zu 7 Kohlenstoffatome.

Besonders bevorzugt werden durch das erfindungsgemäße Verfahren Verbindungen der Formel (I) in welcher

$R^1$ für Alkyl mit bis zu 4 Kohlenstoffatomen steht und

$R^2$ und $R^3$ gleich oder verschieden sind und für Alkyl mit bis zu 4 Kohlenstoffatomen, Alkoxy mit bis zu 4 Kohlenstoffatomen, Phenyl, Benzyl, Phenoxy, Benzyloxy, Benzoyl, Acetyl, Trifluormethyl, Trifluormethoxy, Nitro, Fluor, Chlor, Brom, Hydroxy, Amino, Dimethylamino, Diethylamino, Acetylamino, Carboxy oder Sulfo steht mit der Ausnahme, daß $R^1$ nicht Methyl bedeutet, wenn $R^2$ und $R^3$ Wasserstoff bedeuten, in Form ihrer Isomeren, Isomerengemische, Racemate oder optischen Antipoden hergestellt.

Verwendet man z. B. (±) 4(S*), 5(R*), 7(S*)-5-α-Hydroxybenzyl-1-methyl-4-phenylpyrrolidin-2-on als Ausgangsstoff, läßt sich der Reaktionsablauf durch folgendes Schema verdeutlichen:

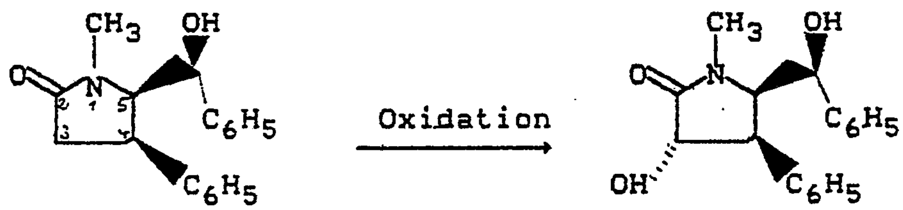


Als Oxidationsmittel können organische oder anorganische Peroxoverbindungen wie beispielsweise Peroxoessigsäure, Chlorperbenzoesäure oder Molybdänperoxid/Pyridin-Komplex, außerdem Sauerstoff, Ozon oder Sauerstoffüberträger wie beispielsweise 2-Sulfonyloxaziridin eingesetzt werden.

Als Lösungsmittel kommen die üblichen inerten organischen Lösungsmittel in Frage, die sich unter den Reaktionsbedingungen nicht verändern. Hierzu gehören vorzugsweise Kohlenwasserstoffe wie beispielsweise Benzol, Toluol, Xylol, Hexan, Cyclohexan oder Erdölfraktionen, Ether wie beispielsweise Diethylether, Tetrahydrofuran oder Dioxan, Alkohole wie beispielsweise Methanol, Ethanol, Isopropanol oder Propanol, Halogenkohlenwasserstoffe wie beispielsweise Dichlormethan, Trichlormethan, Tetrachormethan oder 1,2-Dichlorethan, oder Eisessig oder Hexamethylphosphorsäuretriamid. Ebenso ist es möglich, Gemische der genannten Lösungsmittel zu verwenden.

Als Basen können die zur Enolatbildung üblichen Basen verwendet werden. Hierzu gehören bevorzugt Alkalialkoholate, Alkaliamide, Alkalihydride oder Alkaliorganoverbindungen wie beispielsweise Natrium- bzw. Kaliummethanolat, Natrium- bzw. Kaliumethanolat, Kalium-tert.-butanolat, Natriumhydrid, Natriumamid, Lithiumdiisopropylamid, Butyllithium oder Phenyllithium. Ebenso können teriäre Amine wie beispielsweise 1,5-Diazabicyclo(4,3,0)non-5-en oder 1,8-Diazabicyclo(5,4,0)undec-7-en eingesetzt werden. Besonders bevorzugte Basen sind Lithiumdiisopropylamid, Lithiumhexamethylpiperidid sowie n-, sec- oder tert.-Butyllithium und Phenyllithium.

Die Wahl der Base, des Lösungsmittels sowie gegebenenfalls des Hilfsstoffes richtet sich nach der ausgewählten Oxidationsmethode.

Als Hilfsstoffe werden gegebenenfalls Stoffe eingesetzt, die in situ entstehende Hydroperoxidzwischenstufen zu reduzieren vermögen, insbesondere bei der Verwendung von Molybdänperoxid/Pyridin oder Sauerstoff als Oxidationsmittel. Bevorzugt werden hierfür Phosphite verwendet, insbesondere Trialkyl- oder Triarylphosphite wie beispielsweise Trimethylphosphit, Triethylphosphit, Tripropylphosphit, Triisopropylphosphit, Tributylphosphit oder Triphenylphosphit.

Besonders geeignet ist die Oxidation mit Molybdänperoxid/Pyridin in Hexamethylphosphorsäuretriamid sowie mit Sauerstoff, jeweils unter Verwendung von Phosphiten als Hilfsstoff. Ganz besonders gute Ausbeuten erhält man bei der Oxidation mit Sauerstoff in einem Lösungsmittel wie Tetrahydrofuran oder Hexamethylphosphorsäuretriamid, gegebenenfalls Gemische derselben, unter Verwendung von Triethylphosphit als Hilfsstoff. Es hat sich hierbei als günstig erwiesen, Lithiumdiisopropylamid oder Butyllithium als Base zu verwenden.

Die Reaktionstemperaturen können zwischen -100°C und +20°C variiert werden. Bevorzugt wird in einem Temperaturbereich von -80°C bis 0°C gearbeitet.

Die Hydroxylierung nach dem erfindungsgemäßen Verfahren kann bei normalem, aber auch bei erhöhtem oder erniedrigten Druck durchgeführt werden. Im allgemeinen arbeitet man bei Normaldruck.

Bei der Durchführung des erfindungsgemäßen Verfahrens werden pro Mol der Ausgangsverbindung 1 bis 5, bevorzugt 1 bis 2,5 Mol Base sowie 0,5 bis 5, bevorzugt 0,5 bis 2 Mol des Hilfsstoffes eingesetzt.

Üblicherweise stellt man zunächst in dem geeignetsten Lösungsmittel mit Hilfe der Base das Enolat von (II) her und leitet durch die Lösung unter Zusatz von Phosphit solange absoluten Sauerstoff, bis dünnschichtchromatographisch keine Veränderung mehr zu erkennen ist. Die Aufarbeitung des Reaktionsgemisches erfolgt in üblicher, dem Fachmann vertrauter Weise.

Die Ausgangsverbindungen der Formel (II) sind neu. Sie können hergestellt werden, indem man Aldehyde der allgemeinen Formel (III)

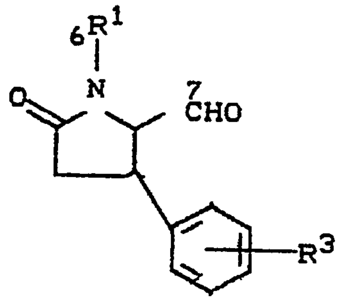

(III),

in welcher
$R^1$ und $R^3$ die oben angegebene Bedeutung haben,
mit Verbindungen der allgemeinen Formel (IV)

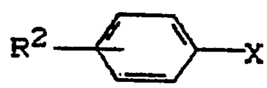

(IV)

in welcher
$R^2$ die oben angegebene Bedeutung hat und
X für -MgBr, -MgCl, -Li oder für $-Ti[OCH(CH_3)_2]_3$ steht,
in geeigneten inerten organischen Lösungsmitteln in einem Temperaturbereich von -20°C bis +50°C, bevorzugt von -10°C bis +30°C umsetzt und das Produkt gegebenenfalls am Kohlenstoffatom 7 epimerisiert.

Besonders geeignet sind Verbindungen der Formel (IV), in welcher X für -MgCl, -MgBr oder $-Ti[OCH(CH_3)_2]_3$ steht.

Als Lösungsmittel eignen sich alle inerten organischen Lösungsmittel, die bei Reaktionen mit metallorganischen Reagentien üblicherweise verwendet werden. Hierzu gehören bevorzugt Ether wie Diethylether oder Tetrahydrofuran, gegebenenfalls vermischt mit Hexan.

Die Umsetzung kann in Analogie zu literaturbekannten Verfahren durchgeführt werden, wie sie beispielsweise von D. Seebach, B. Weidmann und L. Widler in "Modern Synthetic Methods 1983", S. 217 ff (Verlag Salle und Sauerländer) oder in Houben Weyls "Methoden der organischen Chemie" Band XIII/2a, S. 289 ff, S. 302 ff oder von N. L. Drake und G. B. Woke in Organic Synthesis, Coll. Vol. II, 406 ff (1963) beschrieben sind.

Bei der Umsetzung können die Verbindungen der Formel (III) in Form ihrer Isomeren, Isomerengemische, Racemate oder optischen Antipoden eingesetzt werden. Bevorzugt werden die an C(4)-C(5)-cis-konfigurierten Verbindungen der Formel (III) eingesetzt.

Je nach Art des verwendeten metallorganischen Reagenzes können zunächst die am Kohlenstoffatom 7 R-konfigurierten Verbindungen der allgemeinen Formel (IIa)

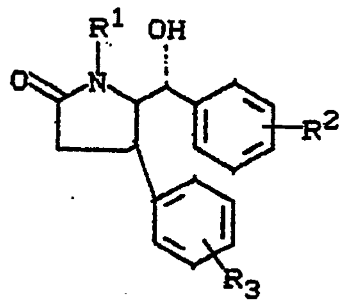

(IIa)

in welcher $R^1$, $R^2$ und $R^3$ die oben angegebene Bedeutung haben,
entstehen,
die durch Oxidation zu Verbindungen der allgemeinen Formel (V)

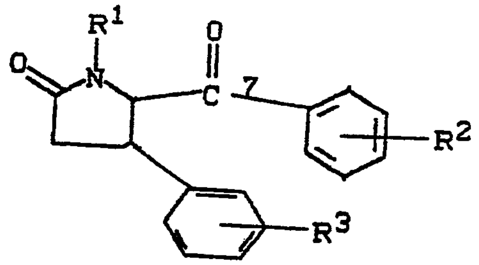

(V)

in welcher $R^1$, $R^2$ und $R^3$ die oben angegebene Bedeutung haben,
und anschließende Reduktion von (V) zu 7-S-konfigurierten Verbindungen der allgemeinen Formel (IIb)

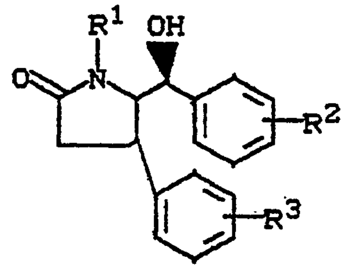

(IIb)

in welcher $R^1$, $R^2$ und $R^3$ die oben angegebene Bedeutung haben,
epimerisiert werden.

Die Oxidation von (IIa) zu (V) erfolgt in Analogie zu bekannten Verfahren mit Dimethylsulfoxid als Oxidationsmittel, unter Zugabe von Anhydriden, insbesondere von Trifluoracetanhydrid, in geeigneten organischen Lösungsmitteln, insbesondere in Halogenkohlenwasserstoffen wie z. B. Dichlormethan, Chloroform oder Kohlenwasserstoffen wie Benzol, Toluol, Xylol oder Hexan, oder in Ethern wie Diethylether, Dioxan oder Tetrahydrofuran oder aber in Gemischen der genannten Lösungsmittel, wie es z. B. von S. L. Huang, K. Omura und D. Swern in Synthesis 1980, 297 beschrieben wird.

Die Reduktion von (V) zu (IIb) kann mit den üblichen Reduktionsmitteln durchgeführt werden. Besonders geeignet sind hierfür Metallhydride und komplexe Metallhydride wie beispielsweise Lithiumboranat, Lithiumhydridoborate, Natriumhydridoborate, Borane, Natriumhydridoaluminate, Lithiumhydridoaluminate oder Zinnhydride. Besonders bevorzugt werden Lithiumhydridoborate wie z. B. Lithium-hydrido-triethylborat oder Lithium-hydrido-tris(1-methylpropyl)borat, oder Natriumborhydrid eingesetzt.

Als Lösungsmittel eignen sich die üblichen, bei Reduktionen mit Hydriden verwendeten inerten organischen Lösungsmittel. Bevorzugt sind dies Ether wie Diethylether und Tetrahydrofuran. Die Reduktion wird in Analogie zu bekannten Methoden durchgeführt [W. Friedrichsen in Houben-Weyl's "Methoden der organischen Chemie" VIII/1b, 145 ff; H. C. Brown, S. Krishnamurthy, Chem. Commun. 1972, 868; A. Hajos in Houben-Weyl's "Methoden der organischen Chemie" IV/1d, 267 ff.].

Außerdem können die Verbindungen (IIa) auch in Analogie zu anderen bekannten Verfahren epimerisiert werden, wie z. B. von O. Mitsunobu in Synthesis 1981, 1 ff beschrieben.

Verwendet man z. B. (±)-(4S*), (5R*)-5-Formyl-1-methyl-4-phenylpyrrolidin-2-on als Ausgangsprodukt, kann die Reaktion durch folgendes Schema dargestellt werden:

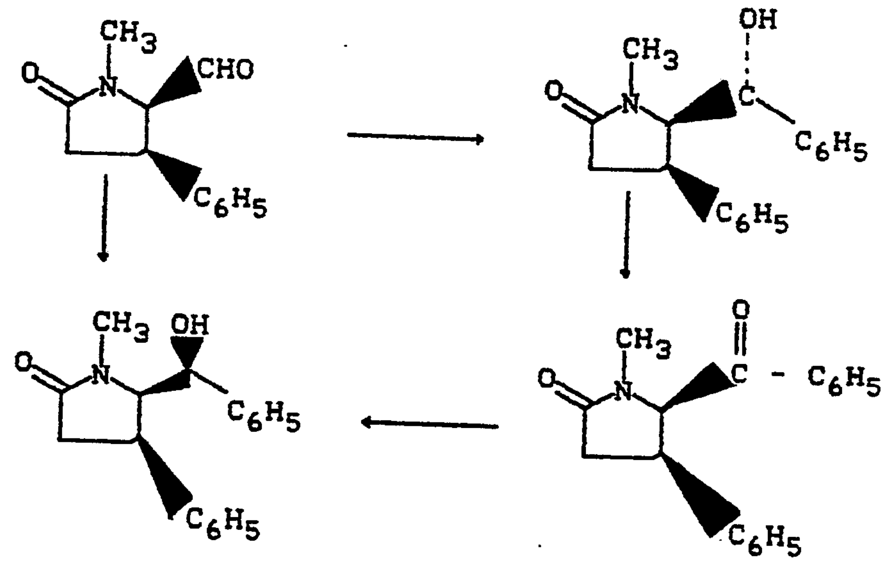

Verwendet man als metallorganische Reagenzien die leicht zugänglichen Grignardverbindungen (X in IV steht für MgCl, MgBr), entstehen ausschließlich die 7-R-konfigurierten Verbindungen IIa, die in angegebener Weise epimerisiert werden können. Sowohl die 7-R- als auch die 7-S-konfigurierten Verbindungen sowie deren Isomere, Isomerengemische, Racemate und optischen Antipoden können erfindungsgemäß zu Verbindungen I hydroxyliert werden.

Die metallorganischen Verbindungen der Formel IV sind bekannt oder können nach bekannten Methoden hergestellt werden [K. Nützel in Houben-Weyl's "Methoden der organischen Chemie" XIII/2a 47 ff].

Die Aldehyde der allgemeinen Formel (III) sind neu und können gemäß folgendem Schema hergestellt werden:

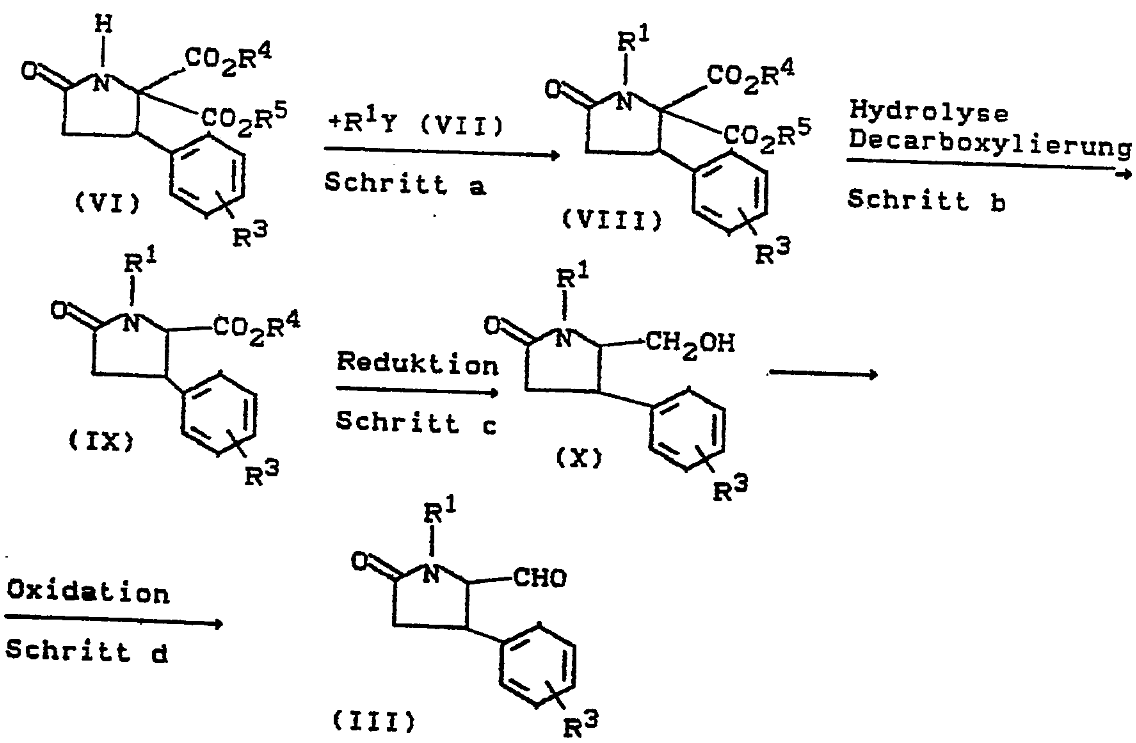


($R^1$, $R^3$ haben die oben angegebene Bedeutung, $R^4$, $R^5$ sind gleich oder verschieden und stehen für $C_1$-$C_4$-Alkyl.)

Danach werden in Schritt a) Verbindungen der allgemeinen Formel (VI) mit Verbindungen der allgemeinen Formel (VII)

$R^1$ - Y (VII),

in welcher
$R^1$ die oben angegebene Bedeutung hat und
Y für Halogen, bevorzugt Brom und Iod, für eine Diazogruppe oder für die Gruppe der Formel

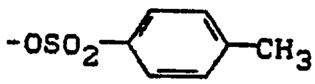 oder $-OSO_2OCH_3$,

oder $-OSO_2OCH_3$, bevorzugt für Iod steht,
gegebenenfalls in Anwesenheit einer Base wie Natrium, Natriumhydrid, Natriumamid, Butyllithium oder Lithiumdiisopropylamid in geeigneten Lösungsmitteln wie Diethylether, Tetrahydrofuran, Dimethylformamid oder Hexamethylphosphorsäuretriamid bei Temperaturen von -20°C bis + 80°C, bevorzugt von 0°C bis +40°C umgesetzt. Ganz besonders geeignet ist Dimethylformamid als Lösungsmittel. Es hat sich hierbei als günstig erwiesen, Natriumhydrid als Base zu verwenden. Durchführung und Aufarbeitung erfolgt nach üblichen, dem Fachmann geläufigen Methoden.

Im Schritt b) werden die Verbindungen der allgemeinen Formell VIII (erhalten aus VI) in Analogie zu dem von P. Pachaly in Chem. Ber. 104 (2), 421-39 (1971) beschriebenen Verfahren hydrolysiert und decarboxyliert und das dabei anfallende Isomerengemisch von (IX) (C(4)-C(5)-cis oder -trans) gegebenenfalls durch allgemein bekannte Methoden der Chromatographie oder durch Umkristallisieren getrennt. Die weiteren Umsetzungen können sowohl mit dem Isomerengemisch als auch mit den einzelnen cis- bzw. trans-Isomeren durchgeführt werden. Bevorzugt wird das Gemisch IX getrennt und mit den einzelnen Isomeren weitergearbeitet, bevorzugt mit den C(4)-C(5)-cis konfigurierten Isomeren.

Die Reduktion der Verbindungen der Formel IX zu Verbindungen der Formel X (Schritt c) erfolgt nach der gleichen Methode und unter der gleichen Bedingung, wie bereits für die Reduktion der Verbindungen (V) zu den Verbindungen (IIb) angegeben.

Die Oxidation von Verbindungen der Formel X zu Verbindungen der Formel III (Schritt d) erfolgt nach den gleichen Methoden und unter den gleichen Bedingungen, wie bereits für die Oxidation von Verbindungen der Formel (IIa) zu Verbindungen der Formel (V) angegeben.

Die Ausgangsverbindungen der Formel VI sind literaturbekannt oder können nach literaturbekannten Methoden hergestellt werden [G. H. Cocolas, W. H. Hartung, J. Am. Chem. Soc. 79, 5203 (1957); F. Zymalkowski, P. Pachaly, Chem. Ber. 100, 1137 (1967)].

In folgendem Schema soll die gesamte Synthese der Endprodukte der Formel (I) mit allen möglichen Zwischenverbindungen (II) - (X) verdeutlicht werden:

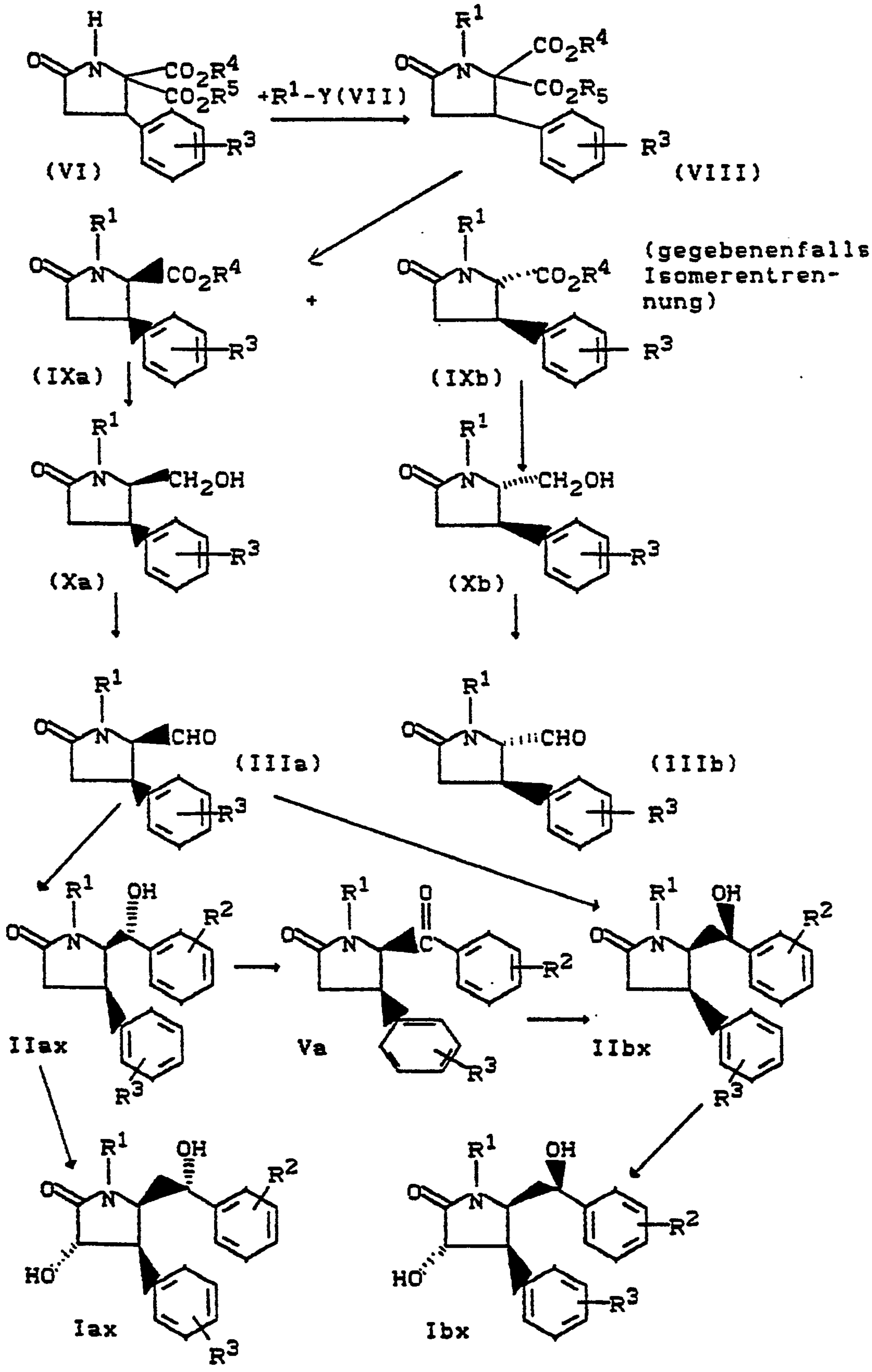


(VI) (VIII)

(IXa) (IXb)

(Xa) (Xb)

(IIIa) (IIIb)

IIax Va IIbx

Iax Ibx

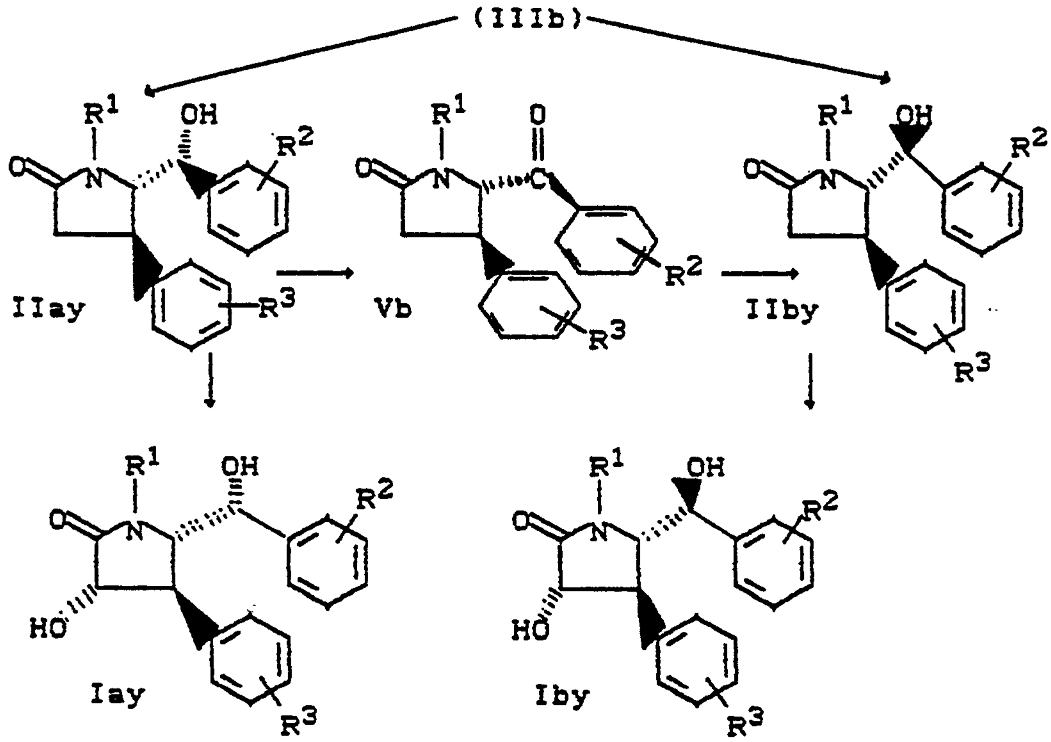
(IIIb)

IIay Vb IIby

Iay Iby

Herstellungsbeispiele

Beispiel 1

(±) 5,5-Diethoxycarbonyl-4-phenylpyrrolidin-2-on

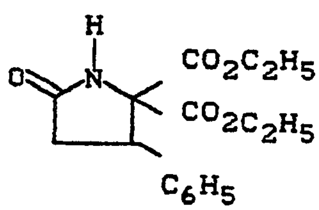

Zur Suspension von 432 g (2 mol) Acetamidomalonsäurediethylester in 1.6 l absolutem Ethanol tropfte man bei Raumtemperatur unter $N_2$-Atmosphäre eine Lösung von 18 g (0.8 Grammatom) Natrium in 400 ml absolutem Ethanol. Man gab 564 g (3.2 mol) Zimtsäureethylester langsam zu und erhitzte anschließend 24 h zum Sieden.

Zur Aufarbeitung ließ man auf Raumtemperatur kommen, gab 2.5 l Chloroform zu und neutralisierte mit Essigsäure. Die Mischung wurde gut mit Wasser gewaschen (5 x je 500 ml), über $MgSO_4$ getrocknet und einrotiert. Der ölige Rückstand wurde in wenig Aceton gelöst, mit Hexan bis zur Kristallisation versetzt und anschließend weiter Hexan zugegeben, bis an der Eintropfstelle keine Trübung mehr zu beobachten war. Absaugen lieferte 398 g (54 %) der Titelverbindung vom Schmelzpunkt 97-99°C. Chromatographie der Mutterlauge (Toluol/Essigester) ergab weitere 85 g (14 %) der Titelverbindung, Gesamtausbeute 413 g (68%).

IR(KBr): ν = 1770 (Ester), 1700 (Amid)

$^1$H-NMR (300 MHz, $CDCl_3$): δ = 0.84 und 1.28 (je t, J=7.5 Hz; 6 H, $CH_2$<u>$CH_3$</u>); ABX Signal: $\delta_A$ = 2.63, $\delta_B$ = 2.96 ($J_{AB}$ = 17.3 Hz, $J_{AX}$ = 6 Hz, $J_{BX}$ = 9 Hz; 2 H, C(3)-H); 3.66 und 3.71 (je m, 2 H, cis-<u>$CH_2$</u>$CH_3$); 4.28 (m, 2H, <u>trans-$CH_2$</u>$CH_3$); 4.39 (dd, $J_{AX}$ = 6 Hz, $J_{BX}$ = 9 Hz, 1H, C(4)-H); 6.95 (br, 1H, NH); 7.39 (br, 5H, $C_6H_5$).

Beispiel 2

(±) 5,5-Diethoxycarbonyl-1-methyl-4-phenyl-pyrrolidin-2-on

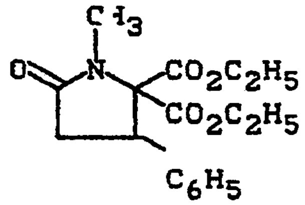

Zur Suspension von 9.64 g (0.36 mol) Natriumhydrid in 200 ml absolutem Dimethylfomamid tropfte man bei Raumtemperatur unter $N_2$-Atmosphäre die Lösung von 100 g (0.33 mol) (±) 5,5-Diethoxycarbonyl-4-phenylpyrrolidin-2-on in 500 ml absolutem Dimethylformamid. Man ließ so lange bei Raumtemperatur nachrühren, bis die Gasentwicklung beendet war, gab anschließend die Lösung von 93.7 g (0.66 mol) Methyliodid in 50 ml absolutem Dimethylformamid zu und rührte bei Raumtemperatur bis alles Ausgangsmaterial umgesetzt war (ca. 1 h, DC Kontrolle). Man goß die Reaktionsmischung in 2 l Pufferlösung pH = 7 und extrahierte fünfmal mit je 600 ml Diethylether.

Trocknen der organischen Extrakte ($MgSO_4$) und Abziehen des Solvens im Vakuum ergaben 105 g (99.6%) der Titelverbindung (nach $^1$H-NMR Spektrum zu 95 % rein) die direkt weiter umgesetzt wurden. Zur Analyse wurde eine Probe am Kugelrohr destilliert ($Kp_{0.5}$ : 240°C) $R_f$: 0.36 (Toluol/Essigester: 2/1),
IR (Film): ν = 1735 (Ester), 1700 (Amid)
$^1$H-NMR (500 MHz, $CDCl_3$): δ = 0.9 und 1.33 (je t, J=7.5 Hz; 6 H, $CH_2\underline{CH_3}$); ABX Signal: $δ_A$ = 2.66, $δ_B$ = 3.0 ($J_{AB}$ = 18 Hz, $J_{AX}$ = 6 Hz, $J_{BX}$ = 8.3 Hz; 2 H, C(3)-H); 3.06 (s; 3H, N-CH); 3.62 und 3.79 (je m, 2 H, cis-$\underline{CH_2}CH_3$); 4.31 (m, 3H, trans-$\underline{CH_2}CH_3$) und C(4)-H); 7.26 (m, 5H, $C_6H_5$).

Beispiel 3

(±)-4(R*), 5(R*) [I] und (±)-4(R*), 5(S*)-5-Ethoxycarbonyl-1-methyl-4-phenylpyrrolidin-2-on [II]

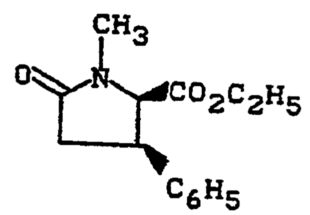

+

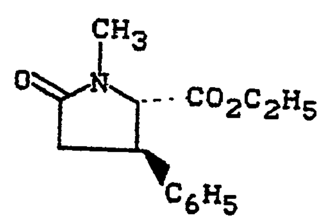

[I] [II]

49.5 g (0.156 Mol) Bariumhydroxidoctahydrat wurden in 483 ml destilliertem Wasser auf 70°C bis zur nahezu klaren Lösung erwärmt. Man gab die Lösung von 100 g (0.313 Mol) (±)5,5-Diethoxycarbonyl-1-methyl-4-phenyl-pyrrolidin-2-on in 724 ml Ethanol zu (klare Lösung) und rührte 20 min. bei 70°C nach, bis das Ausgangsmaterial vollständig umgesetzt war (ca. 20 Minuten, DC-Kontrolle). Man kühlte ab, säuerte unter Eiskühlung auf pH = 1-2 an und zog das Ethanol im Vakuum ab (Badtemperatur 30-40°C). Man saugte den Festkörper ab und extrahierte wäßrige Phase unter Kochsalz-Zusatz 3 mal mit je 200 ml Essigester. Trocknen und Abziehen des Solvens lieferte einen Rückstand, der mit dem oben erhaltenen Festkörper vereinigt und 24 h in Exsikkator über $P_4O_{10}$ im Hochvakuum getrocknet wurde. Anschließend wurde der Festkörper unter gutem Rühren im Ölbad auf 170°C erwärmt, bis die Gasentwicklung beendet war (5-10 Minuten). Abkühlen und flash-Chromatographie (Cyclohexan/Essigester = 1/1, zum Schluß mit Essigester) lieferten 39.3 g (50.7 %) des cis-Produktes I mit $R_f$ = 0.10 und 19.6 g (25.3 %) des trans-Produktes II mit $R_f$ = 0.20 (jeweils in Cyclohexan/Essigester 1/1).
IR(KBr): ν = 1736, 1690 cm$^{-1}$
$^1$H-NMR (200 MHz, $CDCl_3$) [I]: δ = 0.83 (t, J = 7.5 Hz, 3H, $CH_2\underline{CH_3}$) ABX Signal: $δ_A$ = 2.67, $δ_B$ = 2.95 ($J_{AB}$ = 17.5 Hz, $J_{AX}$ = 9 Hz, $J_{BX}$ = 10 Hz, 2 H, C(3)-H), 2.87 (s, 3H, N-$CH_3$), 3.75 (m, 2H, $\underline{CH_2}$CH3); 3.91 (q, J = 9-10 Hz, 1H, C(4)-H), 4.36 (d, J = 9 Hz, 1 H, C(5)-H), 7.28 (m, 5H, $C_6H_5$).
[II]: δ = 1.30 (t, J = 7.5 Hz, 3H, $CH_2\underline{CH_3}$); ABX-Signal: δA = 2.54, δB = 2.82 ($J_{AB}$ = 18.5 Hz, $J_{AX}$ = 5 Hz, $J_{BX}$ = 9 Hz, 2H, C(3)-H), 3.80 (s, 3H, N-$CH_3$), 3,53 (ddd, J = 9 Hz, J = 5 Hz, J = 4 Hz, 1H, C(4)-H), 4.07 (d, J = 4 Hz, 1H, C(5)-H), 4.27 (m, 2H, $CH_2$-$CH_3$) 7.3 (m, 5H, $C_6H_5$).

Beispiel 4

(±) 4(R*), 5(R*)-5-Hydroxymethyl-1-methyl-4-phenylpyrrolidin-2-on

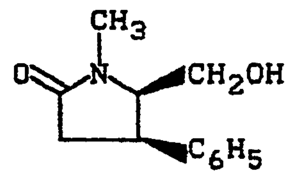

Zur Lösung von 39.2 g (0.159 mol) (±) 4(S*), 5(R*)-5-Ethoxycarbonyl-1-methyl-4-phenylpyrrolidin-2-on in 390 ml absolutem Tetrahydrofuran tropfte man bei -15 bis -20°C unter $N_2$-Atmosphäre 0.317 mol $LiB(Et)_3H$ (als 1 M-Lösung in Tetrahydrofuran, 316.9 ml).

Man ließ 1 h bei 0°C nachrühren, goß die Reaktionsmischung in ca. 200 ml eiskalte 2N Salzsäure und extrahierte zweimal mit je 200 ml Essigester. Man sättigte die wäßrige Phase mit Kochsalz und extrahierte nochmals zweimal mit je 200 ml Essigester. Die gesammelten organischen Extrakte wurden mit wenig Wasser gewaschen, über $MgSO_4$ getrocknet und einrotiert. Der Rückstand wurde mit wenig Ether zur Kristallisation gebracht und anschließend mit Pentan ausgefällt, bis an der Eintropfstelle keine Trübung mehr zu beobachten war. Nach Absaugen und Trocknen erhielt man 29.1 g (89.2%) der Titelverbindung mit Schmelzpunkt 93-95°C.

IR(KBr): $\nu$ = 3324, 1687 $cm^{-1}$

$^1H$-NMR (300 MHz, $CDCl_3$): $\delta$ = AB-Teil von ABM System, $\delta_A$ = 2.59, $\delta_B$ = 2.97 (je dd, $J_{AB}$ = 15 Hz, $J_{AM}$ = 7.5 Hz, $J_{BM}$ = 9 Hz, 2H, C(3)-H); 2.97 (s, 3H, $N-CH_3$) AB-Teil von ABM-System, $\delta_A$ = 3.36, $\delta_B$ = 3.62 (je dd, $J_{AB}$ = 11.2 Hz, $J_{AM}$ = $J_{BM}$ = 3 Hz, 2H, C(7)-H); 3.72 - 3.85 (m, 2H, C(4)-H, C(5)-H); 7.32 (m, 5H, $C_6H_5$).

Beispiel 5

(±)4(R*), 5(R*)-5-Formyl-1-methyl-4-phenylpyrrolidin-2-on

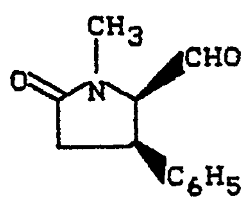

Zur Lösung von 19.9 ml (0.28 Mol) absolutem Dimethylsulfoxid in 140 ml absolutem Dichlormethan tropfte man unter $N_2$-Atmosphäre innerhalb 10 min bei -60°C die Lösung von 29.7 ml Trifluoracetanhydrid in 56 ml absolutem Dichlormethan. Man ließ 15 min bei dieser Temperatur rühren und tropfte die Lösung von 28.8 g (0.140 Mol) (±) 4(S*), 5(S*)-5-Hydroxymethyl-1-methyl-4-phenylpyrrolidin-2-on in 250 ml Dichlormethan so zu, daß die Temperatur -60°C nicht überstieg. Man ließ 90 min bei -60°C nachrühren, erwärmte kurz auf -30°C (5-10 min) und kühlte wieder auf -60°C ab. Man gab langsam bei dieser Temperatur 56 ml absolutem Triethylamin zu, ließ 30 min bei -60°C rühren und erwärmte auf Raumtemperatur. Man gab 600 ml Wasser zu, trennte die Phasen und extrahierte die wäßrige Phase dreimal mit je 250 ml Dichlormethan. Die gesammelten organischen Extrakte wurden zweimal mit je 300 ml Wasser gewaschen, über Magnesiumsulfat getrocknet und extrahiert. Man erhielt 28.3 g der Titelverbindung mit $R_f$ = 0.25 (Essigester) (nach $^1H$-NMR-Spektrum zu 91 % rein). Das so erhaltene Rohprodukt wurde nach Trocknung (24 h, HV) direkt weiter umgesetzt.

IR ($CHCl_3$): $\nu$ = 1734, 1689 $cm^{-1}$

$^1H$-NMR (300 MHz, $CDCl_3$): $\delta$ 2.79 (dd, J=5.3 Hz, J=9.7 Hz, 2H, C(3)-H); 2.91 (s, 3H, $N-CH_3$); 4.02 (q, J=9.7 Hz, 1H, C(4)-H); 4.30 (dd, J=1 Hz, J=9.7 Hz, 1H, C(5)-H); 7.3 (m, 5H, $C_6H_5$, 9.17 (d, J=1 Hz, 1H, CHO).

Beispiel 6

(±) 4(R*), 5(R*), 7(R*)-5-α-Hydroxybenzyl-1-methyl-4-phenylpyrrolidin-2-on

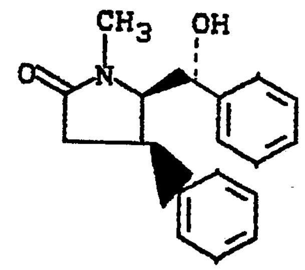

Zu 3.84 g Mg-Späne tropfte man unter $N_2$ die Lösung von 24.8 g (16.7 ml, 0.156 Mol) Brombenzol in 44 ml absolutem Tetrahydrofuran so zu, daß das Tetrahydrofuran gelinde siedete. Man gab anschließend 100 ml abs. Tetrahydrofuran zu und erhitzte unter Rückfluß zum Sieden bis alles Magnesium gelöst war (1-2 h).
Man kühlte auf 0°C und tropfte unter kräftigem Rühren die Lösung von 24,7 g (0,12 Mol) (±) 4(S*), 5(R*)-5-Formyl-1-methyl-4-phenylpyrrolidin-2-on in 250 ml absolutem Tetrahydrofuran so zu, daß die Temperatur 5°C nicht überstieg. Gegebenenfalls mußte absolutem Tetrahydrofuran zur besseren Rührfähigkeit zugegeben werden. Man rührte anschließend 1 h bei 0-5°C und goß die Reaktionsmischung auf 350 ml 0.5N HCl-Eis und extrahierte viermal mit je 300 ml Essigester und zweimal mit je 300 ml Dichlormethan. Die gesammelten Essigester- und Dichlormethan-Extrakte wurden (getrennt!) zweimal mit je 200 ml Wasser gewaschen, vereinigt und über Magnesiumsulfat getrocknet. Der nach Abziehen des Solvens (im Vakuum) verbleibende Rückstand wurde mit 100 ml Ether bis zur Kristallisation verrieben. Anschließend gab man 500 ml Pentan langsam zu und ließ über Nacht im Kühlschrank stehen. Absaugen des Festkörpers ergab 25 g (74.3 %) der Titelverbindung mit Schmelzpunkt: 210-212°C.
Zur Analyse wurde aus Aceton umkristallisiert (Schmelzpunkt : 214-5°C).
IR(KBr) ν = 3362 (br), 1654 $cm^{-1}$
$^1$H-NMR (300 MHz, $d_6$-DMSO): δ = 2.21 (s, 3H, $NCH_3$); 2.24 (dd, A-Teil von ABM-System, $J_{AB}$=15.7 Hz, $J_{AM}$=9.4 Hz, 1H, cis-C(3)-H); 3.05 (dd, B-Teil von ABM-System, $J_{BM}$=12.7 Hz, 1H, trans-C(3)-H); 3.80 (dt, M-Teil von ABM-System, $J_{AM}$=8.5 Hz, $J_{AB}$=12.7 Hz, $J_{4,5}$=8.5 Hz, 1H, C(4)-H); 4,15 (dd, J=8.5 Hz, J=1 Hz, 1H, C(5)-H); 4.26 (dd, J=6 Hz, J=1 Hz, 1H, C(7)-H); 5.35 (d, J=6 Hz, 1H, OH); 7.15-7.5 (m, 1OH, $C_6H_5$).

Beispiel 7

(±)4(R*), 5(R*)-5-Benzoyl-1-methyl-4-phenylpyrrolidin-2-on

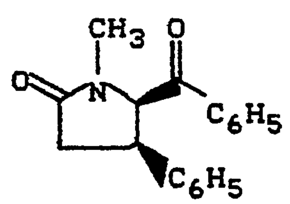

Zur Lösung von 12.24 ml (0.171 Mol) absolutem Dimethylsulfoxid in 87 ml absolutem Dichlormethan tropfte man unter $N_2$-Atmosphäre innerhalb 10 min bei -60°C die Lösung von 18 ml Trifluoracetanhydrid in 34 ml absolutem Dichlormethan. Man ließ 15 min bei dieser Temperatur nachrühren und tropfte die Lösung von 24 g (0.085 Mol) (±)4(S*), 5(S*), 7(S*)-5-α-Hydroxybenzyl-1-methyl-4-phenylpyrrolidin-2-on in ca. 700 ml absolutem Dichlormethan so zu, daß die Temperatur -60°C nicht überstieg. Man ließ 90 min bei -60°C nachrühren, erwärmte kurz auf -30°C (9-10 min) und kühlte wieder auf -60°C ab. Man gab langsam bei dieser Temperatur 34.2 ml Triethylamin zu, ließ 20 min bei -60°C rühren und erwärmte auf Raumtemperatur. Man gab 370 ml Wasser zu, trennte die Phasen und extrahierte die wäßrige Phase dreimal mit je 250 ml Dichlormethan. Die vereinigten organischen Extrakte wurden zweimal mit je 300 ml Wasser gewaschen, über Magnesiumsulfat getrocknet und einrotiert. Der Rückstand wurde zweimal mit je 200 ml Ether abrotiert. Man erhielt 23.5 g (100 %) der Titelverbindung als Festkörper mit Schmelzpunkt: 115-116°C. Das Rohprodukt, das nach $^1$H-NMR Spectrum rein war, wurde direkt weiter umgesetzt.

Zur Analyse wurde eine Probe über Kieselgel mit Essigester chromatographiert ($R_f$ = 0.25) Schmelzpunkt: 121-2°C
IR(KBr): ν = 1695, 1682 $cm^{-1}$
$^1$H-NMR (300 MHz, $CDCl_3$) δ=2.78 und 2.91 (AB-Teil vom ABM-Spektrum, $J_{AB}$=16.5 Hz, $J_{AM}$=$J_{BM}$=8.3 Hz, 2H, C(3)-H); 2.88 (s, 3H, N-$CH_3$); 4.02 (q, J=8.3 Hz, 1H, C(4)-H); 5.42 (d, J=8.3 Hz, 1H, C(5)-H); 7.0, 7.21, 7.59, 7.50 (je m, 10H, $C_6H_5$).

Beispiel 8

(±)4(R*), 5(R*), 7(S*)-5-α-Hydroxybenzyl-1-methyl-4-phenylpyrrolidin-2-on

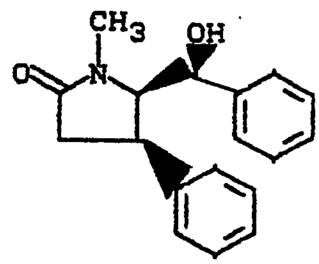

Zur Lösung von 23 g (82.3 mmol) (±) 4(S*), 5(R*)-5-Benzoyl-1-methyl-4-phenylpyrrolidin-2-on in 200 bis 270 ml absolutem Tetrahydrofuran tropfte man bei -15°C bis -20°C unter $N_2$-Atmosphäre 83 mmol $LiB(Et)_3H$ (83 ml einer 1M-Lösung in Tetrahydrofuran). Man ließ 1 h bei 0°C nachrühren, goß die Reaktionsmischung in 100 ml eiskalte 1N HCl und extrahierte zweimal mit je 200 ml Essigester. Man sättigte die wäßrige Phase mit Kochsalz und extrahierte nochmals zweimal mit je 200 ml Essigester. Die vereinigten organischen Extrakte wurden über $MgSO_4$ getrocknet und einrotiert. Den Rückstand löste man in Dichlormethan und wusch zweimal mit je 100 ml Wasser. Die organische Phase wurde getrocknet ($MgSO_4$) und einrotiert. Der Rückstand wurde mit 100 ml Ether zur Kristallisation gebracht und anschließend langsam unter Rühren mit Pentan versetzt bis an der Eintropfstelle keine Trübung mehr zu beobachten war. Der Niederschlag wurde abgesaugt und getrocknet. Man erhielt 16.6 g (72 %) der Titelverbindung mit Schmelzpunkt: 189-195°C.

Das Produkt ist nach $^1H$-NMR zu 95 % rein und wurde direkt weiter umgesetzt.

Zur Analyse wurde aus Aceton umkristallisiert (Schmelzpunkt: 197-8°C).

IR(KBr): $\nu$ = 3251, 1692 $cm^{-1}$

$^1H$-NMR (300 MHz, DMSO): $\delta$ = 1.97 und 2.05 (ABM-Signal, $J_{AB}$=13.5 Hz, $J_{AM}$=8.2 Hz, $J_{BM}$=13 Hz, 2H C(3)-H); 2,91 (s, 3H, $N-CH_3$); 3.82 (dt,$J_{AM}$=$J_{4,5}$=8.2 Hz, $J_{BM}$=13 Hz, 1H, C(4)-H); 4.27 (dd, J=8.2 Hz, J=1.5 Hz, 1H, C(5)-H); 4.65 (dd, J=1.5 Hz, J=3.5 Hz, 1H, C(7)-H); 5.34 (d, J=3.5 Hz, 1H, OH); 6.70, 7.11, 7.25 (je m, 10H, $C_6H_5$).

Beispiel 9

(±)3(S*), 4(R*), 5(R*), 7(S*)-3-Hydroxy-5-α-hydroxybenzyl-1 methyl-4-phenylpyrrolidin-2-on (Clausenamid)

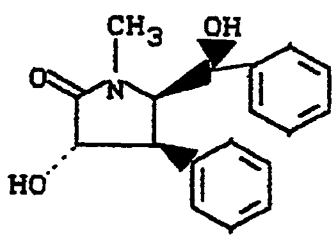

In einem im Vakuum ausgeheizten und mit Reinstsickstoff belüfteten Kolben gab man die Lösung von 17.7 g (62.8 mmol) (±)4(R*), 5(R*), 7(S*)-5-α-Hydroxybenzyl-1-methyl-4-phenylpyrrolidin-2-on in 490 ml absolutem Tetrahydrofuran und 130 ml absolutem Hexamethylphosphorsäuretriamid und kühlte auf -70°C. Bei dieser Temperatur tropfte man die Lösung von 0.152 Mol Lithiumdiisoprepylamid in 180 ml absolutem Tetrahydrofuran/Hexan zu (hergestellt aus 22.1 ml Diisopropylamin in 80 ml Tetrahydrofuran durch Zugabe von 103 ml einer 1.5N Lösung von n-Butyllithium in Hexan bei -20°C bis 0°C). Man rührte 1 h bei -70°C bis -60°C nach, gab 5.3 ml frisch destilliertes Trimethylphosphit (gelöst in wenig absolutem Tetrahydrofuran) zu und leitete (über $H_2SO_4$ und $P_4O_{10}$ getrockneten) absoluten Sauerstoff ein (50-100 ml/ min). Sobald nach DC-Kontrolle ($SiO_2$; EE/MeOH: 2/1; $R_f$= 0.3 für Titelverbindung, $R_f$= 0.37 für Ausgangsverbindung, Anfärben mit Molybdatophosphorsäure Sprühreagenz sich das Verhältnis Produkt/Ausgangsmaterial nicht mehr änderte (2-3 h) goß man unter Eiskühlung auf 600 ml 0.5N HCl und säuerte gegebenenfalls auf pH 3 bis 4 an.

Man trennte die Phasen und extrahierte die wäßrige Phase viermal mit je 300 ml Essigester. Die vereinigten organischen Extrakte wurden dreimal mit je 300 ml Wasser gewaschen und über $MgSO_4$ getrocknet und einrotiert. Man nahm den Rückstand in 50-100 ml Ether auf, rührte bis zur beginnenden Kristallisation und gab langsam unter Rühren soviel Pentan zu, bis an der Eintropfstelle keine Trübung mehr zu

beobachten war. Man ließ über Nacht im Kühlschrank stehen und saugte ab. Man erhielt ca. 17 g eines rohen Festkörpers der neben der Titelverbindung ca. 35-40 % Ausgangsmaterial enthielt. Zur Reinigung wird zweimal aus Methanol umkristallisiert. Man erhält dann die Titelverbindung in ca. 95 % Reinheit. Verlustfreier und mit Rückgewinnung des reinen Ausgangsmaterials verläuft die Chromatographie über Aluminiumoxid (neutral). Hierzu wird das Rohprodukt auf Kieselgel aufgezogen (Lösen in MeOH in der Wärme, Zugabe von 5 Gew.-Teilen Kieselgel, Einrotieren und mehrfaches Abrotieren mit Essigester, bis ein staubtrockenes MeOH-freies Produkt resultiert). Das Adsorbat wird auf eine Säule mit $Al_2O_3$ (neutral, 50 Gew.-Teile) gegeben und mit Essigester zunächst das Ausgangsmaterial eluiert (flash-Chromatographie, Kontrolle mit DC und analyt. HPLC). Anschließend eluiert man die Titelverbindung mit Essigester/Methanol-Gemischen (40/1, 20/1, dann 10/1). Man erhielt 8.6 g (46.1 %) der Titelverbindung mit Schmelzpunkt: 236-7.5°C (auth. Clausenamid: 236-7°C) Reiheit ca. 98 % (nach $^1$H-NMR, enthält ca. 2 % Ausgangsmaterial). 5 g des reinen Ausgangsmaterials konnten zurückgewonnen werden.

IR(KBr): ν = 3402, 3321, 1689 $cm^{-1}$

$^1$H-NMR (300 MHz, DMSO): δ = 3.01 (s, 3H, N-$CH_3$); 3.50 (dd, J=8 Hz, J=10.5 Hz, 1H, C(4)-H); 3.82 (dd, J=10 Hz, J=7 Hz, 1H, C(3)-H); 4.30 (dd, J=8 Hz, J=2 Hz, 1H, C(5)-H); 4.65 (dd, J=2 Hz, J=3 Hz, 1H, C(7)-H), 5.39 (d, J=7 Hz, 1H, C(3)-OH); 5.45 (d, J=3 Hz, 1H, C(7)-OH); 6.61-6.64 (m, 2H, arom. H); 7.03-7.28 (m, 8H, arom. H).

Beispiel 10

(±)3(S*), 4(R*), 5(R*), 7(R*)-3-Hydroxy-5-α-hydroxybenzyl-1-methyl-4-phenylpyrrolidin-2-on (7-epi-Clausenamid)

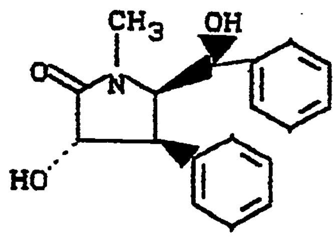

Aus 2 g (0.71 Mol) 4(R*), 5(R*), 7(R*)-5-α-Hydroxybenzyl-1-methyl-4-phenylpyrrolidin-2-on erhielt man nach Ausfällen mit Ether/Pentan 1.8 g eines Festkörpers, der an Kieselgel mit Cyclohexan/Essigester = 1/2 chromatographiert wurde ("flash"-Chromatographie). Man erhielt 0.94 g (45 %) der reinen Titelverbindung mit Schmp. 200-1°C. 0.7 g des Ausgangsmaterials konnten zurückgewonnen werden.

IR(KBr): ν = 3435, 3363, 1660 $cm^{-1}$

$^1$H-NMR (300 MHz, DMSO) δ = 2.18 (s, 3H, N-$CH_3$), 3.54 (dd, J=8 Hz, J=10 HZ, 1H, C(4)-H) 4.12 (d, J=8 Hz, 1H, 1H C(5)-H), 4.25 (d, J=6 Hz, 1H, C(7)-H), 4.93 (dd, J=10 Hz, J=7.5 Hz, 1H, C(3)-H), 5.43 (d, J=6 Hz, 1H, C(3)-OH), 5.47 (d, J=7.5 Hz, 1H, C(7)-OH), 7.18-7.55 (m, 10H, Aromaten H).

Beispiel 11

(±)4(R*),5(R*),7(R*)-5-[1-Hydroxy-1-p-chlorphenyl]-methyl-1-methyl-4-phenylpyrrolidin-2-on

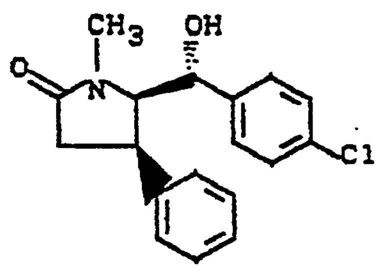

Man verfuhr analog Beispiel 6, in Variation wurde das Grignard-Reagenz im Ultraschallbad hergestellt. Eingesetzt wurden 15,12 g (0,079 mmol) 4-Chlorbrombenzol und 12.19 g (0,06 mol) der Titelverbindung aus Beispiel 5. Nach Aufarbeitung analog Beispiel 6 wurde der Rückstand in wenig heißem Essigester aufgenommen und unter Anreiben mit einem Glasstab langsam abgekühlt (zum Schluß Eisbad). Man ließ 12 h stehen und saugte ab. Man erhielt 11.53 g (60,9% der Theorie) der Titelverbindung vom Schmp.: 201°C (Ether/Pentan).

$^1$H-NMR (200 MHz, $CDCl_3$): δ = 2,44 (s, 3H, $NCH_3$); 2,40 (dd, A-Teil vom ABM-System, $J_{AB}$=15 Hz, $J_{AM}$=9,5 Hz, 1H, cis-C(3)-H); 3,15 (dd, B-Teil vom ABM-System, $J_{BM}$=12,5 Hz, 1H, trans-C(3)-H); 3,78 (dt, M-Teil vom ABM-System, $J_{AM}$=9,5 Hz, $J_{BM}$=12,5 Hz, $J_{4,5}$=7,5 Hz, 1H, C(4)-H); 3,83 (d, J=6 Hz, 1H,

OH); 3,97 (dd, J=7,5 Hz, J=1 Hz, 1H, C(5)-H); 4,39 (dd, J=6 Hz, J=1 Hz, 1H, C(7)-H); 7,12 - 7,38 (m, 9H, Aromaten-H).

Beispiel 12

(±) 4(R*), 5(R*),7(R*)-5-(1-Hydroxy-1-m-fluorophenyl)-methyl-1-methyl-4-phenylpyrrolidin-2-on

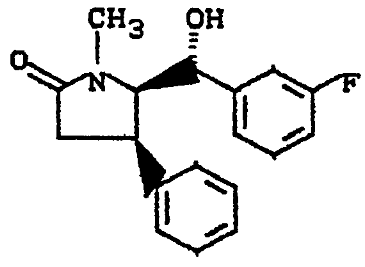

Man verfuhr analog Beispiel 11. Eingesetzt wurden 13,83 g (0,079 mol) 3-Fluorbrombenzol und 12,19 g (0,06 mol) der Titelverbindung aus Beispiel 5. Man erhielt 11.73 g (70,9% der Theorie) der Titelverbindung vom Schmp.:
213°C (Ether/Pentan).
$^1$H-NMR (200 MHz, $CDCl_3$/DMSO): δ = 2,45 (s, 1H, N-$CH_3$); 2,40 (dd, A-Teil vom ABM-System, $J_{AB}$=15 Hz, $J_{AM}$=9,5 Hz, 1H, cis-C(3)-H); 3,20 (dd, B-Teil vom ABM-System, $J_{BM}$=12,5 Hz, 1H, trans-C(3)-H); 3,8 (dt, M-Teil vom ABM-System, $J_{AM}$=9,5 Hz, $J_{BM}$=12,5 Hz, $J_{4,5}$=7,5 Hz, 1H, C(4)-H); 4,03 (dd, J=7,5 Hz, J=1 Hz, 1H, C(5)-H); 4,36 (dd, J=6 Hz, J=1 Hz, 1H, C(7)-H); 5,03 (d, J=6 Hz, 1H, OH); 6,8 – 7,1 und 7,17 – 7,4 (je m, 9H, Aromaten-H).

Beispiel 13

(±) 4(R*), 5(R*), 7(R*)-5-[1-Hydroxy-1-(3-chlorphenyl)]-methyl-1-methyl-4-phenylpyrrolidin-2-on

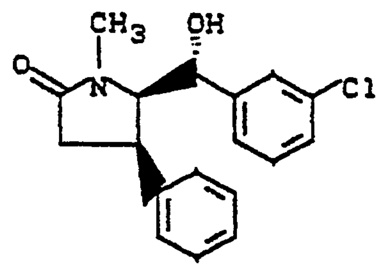

Man verfuhr analog Beispiel 11. Eingesetzt wurden 15,12 g (0,079 mol) 3-Bromchlorbenzol und 12.19 g (0,06 mol) der Titelverbindung aus Beispiel 5. Man erhielt 12,2 g (64,4% der Theorie) der Titelverbindung vom Schmp.: 220°C.
$^1$H-NMR (200 MHz, $CDCl_3$/DMSO): δ = 2,43 (s, 3H, $NCH_3$); 2,38 (dd, A-Teil vom ABM-System, $J_{AB}$=15 Hz, $J_{AM}$=9,5 Hz, 1H, cis-C(3)-H); 3,18 (dd, B-Teil vom ABM-System, $J_{BM}$=12,5 Hz, 1H, trans-C(3)-H); 3,81 (dt, M-Teil vom ABM-System, $J_{AM}$=9,5 Hz, $J_{BM}$=12,5 Hz, $J_{4,5}$=7,5 Hz, 1H, C(4)-H); 4,05 (dd, J=7,5 Hz, J=1 Hz, 1H, C(5)-H); 4,32 (dd, J=6 Hz, J=1 Hz, 1H, C(7)-H); 5,32 (d, J=6 Hz, 1H, OH); 7,12 – 7,45 (m, 9H, Aromaten-H).

Beispiel 14

(±) 4(R*), 5(R*), 7(R*)-5-[1-Hydroxy-1-(2,6-dichlorphenyl)]methyl-1-methyl-4-phenylpyrrolidin-2-on

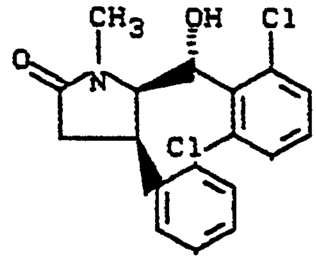

Man verfuhr analog Beispiel 11. Eingesetzt wurden 17,85 g (0,079 mol) 2,6-Dichlorbrombenzol und 12,91 g (0,06 mol) der Titelverbindung aus Beispiel 5. Man erhielt 4,7 g (22,3% der Theorie) der Titelverbindung vom Schmp.: 156°C.
$^1$H-NMR (200 MHz, $CDCl_3$): δ = 2,53 (dd, A-Teil vom ABM-System, $J_{AB}$=17,5 Hz, $J_{AM}$=9,5 Hz, 1H, cis-C(3)-H); 2,92 (s, 3H, N-$CH_3$); 3,08 (dd, B-Teil vom ABM-System, $J_{BM}$=11 Hz, 1H, trans-C(3)-H); 3,50 (d, J=9 Hz, 1H, OH); 3,80 (dt, M-Teil vom ABM-System, $J_{4,5}$=7,5 Hz, 1H, C(4)-H); 4,49 (dd, J=7,5 Hz, J=5 Hz, 1H, C(5)-H); 5,16 (dd, J=9 Hz, J=5 Hz, 1H, C(7)-H); 6,9 – 7,35 (m, 8H, Aromaten-H).

Beispiel 15

(±) 4(R*), 5(R*), 7(R*)-5-[1-Hydroxy-1-(4-fluorophenyl)]-methyl-1-methyl-4-phenylpyrrolidin-2-on

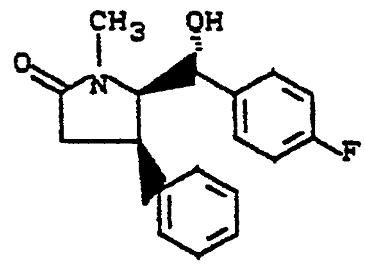

Man verfuhr analog Beispiel 11. Eingesetzt wurden 13,83 g (0,079 mol) 4-Fluorbrombenzol und 12,19 g (0,06 mol) der Titelverbindung aus Beispiel 5. Man erhielt 10,19 g (61,6% der Theorie) der Titelverbindung vom Schmp.: 211-213°C und $R_f$ (Essigsester) = 0,45.
$^1$H-NMR (300 MHz, $CDCl_3$): δ = 2,47 (s, 3H, $NCH_3$); 2,49 (dd, A-Teil vom ABM-System, $J_{AB}$=15,7 Hz, $J_{AM}$=8,2 Hz, 1H, cis-C(3)-H); 2,55 (breit, 1H, OH); 3,20 (dd, B-Teil vom ABM-System, $J_{BM}$=13 Hz, 1H, trans-C(3)-H); 3,82 (dt, M-Teil vom ABM-System,
$J_{4,5}$=7,5 Hz, 1H, C(4)-H); 4,01 (dd, J=7,5 Hz, J=1 Hz, 1H, C(5)-H); 4,48 (d, J=1 Hz, 1H, C(7)-H); 6,95, 7,15, 7,28 und 7,35 (je m, 9H, Aromaten-H).

Beispiel 16

(±) 4(R*),5(R*)-5-(p-Chlorbenzoyl)-1-methyl-4-phenylpyrrolidin-2-on

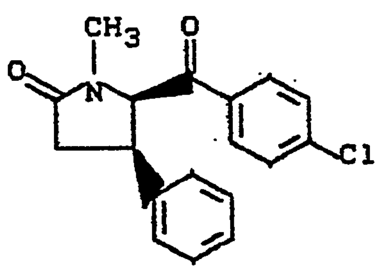

Man verfuhr analog Beispiel 7. Eingesetzt wurden 7,9 g (0,025 mol) der Titelverbindung aus Beispiel 11. Man erhielt 5,72 g (73,1% der Theorie) der Titelverbindung die ohne Reinigung weiterverarbeitet wurden.
$^1$H-NMR (200 MHz, $CDCl_3$); δ = 2,93 (quint, AB-Teil von ABM-Signal, $J_{AB}$=17,5 Hz, $J_{AM}$,$J_{BM}$=9,5 Hz, 2H, C(3)-H); 4,03 (q, M-Teil vom ABM-Signal,
$J_{AM}$=$J_{BM}$=$J_{4,5}$=9,5 Hz, 1H, C(4)-H); 5,39 (d, J=9,5 Hz, 1H, C(5)-H); 7,04 (s, 5H, $C_6H_5$); AB-Signal ($\delta_A$=7,2, $\delta_B$=7,46, $J_{Ab}$=9,5 Hz, 4H, $C_6H_4Cl$).

Beispiel 17

(±) 4(R*),5(R*)-5-m-Fluorobenzoyl-1-methyl-4-phenylpyrrolidin-2-on

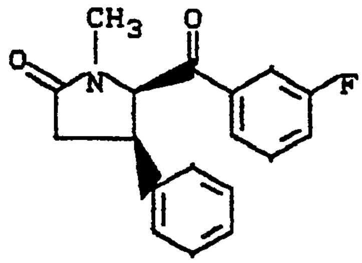

Man verfuhr analog Beispiel 7. Eingesetzt wurden 7,48 g (0,025 mol) der Titelverbindung aus Beispiel 12. Man erhielt 6,36 g (85,6% der Theorie) der Titelverbindung die ohne Reinigung weiterverarbeitet wurden.
$^1$H-NMR (200 MHz, $CDCl_3$): δ = 2,92 (quint, AB-Teil vom ABM-Signal, $J_{AB}$=17,5 Hz,, $J_{AM}$=$J_{BM}$=9,5 Hz, 2H, C(3)-H); 4,01 (q, M-Teil vom ABM-Signal, $J_{AM}$=$J_{BM}$=$J_{4,5}$=9,5 Hz, 1H, C(4)-H); 5,39 (d, J=9,5 Hz, 1H, C(5)-H); 7,03 (s, 5H, $C_6H_5$); 7,05 - 7,35 (m, 4H, $C_6H_4F$).

Beispiel 18

(±) 4(R*),5(R*)-5-p-Fluorobenzoyl-1-methyl-4-phenylpyrrolidin-2-on

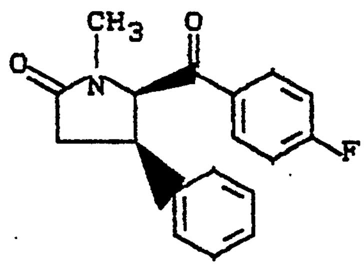

Man verfuhr analog Beispiel 7. Eingesetzt wurden 7,28 g (0,016 mol) der Titelverbindung aus Beispiel 15. Man erhielt 5,78 g (80% der Theorie) der Titelverbindung die ohne Reinigung weiterverarbeitet wurden.
$^1$H-NMR (200 MHz, $CDCl_3$): δ = ABM-Signal ($δ_A$=2,72, $δ_B$=2,89, $J_{AB}$=16 Hz, $J_{AM}$=9,5 Hz, 2H, C(3)-H); 2,88 (s, 3H, $NCH_3$); 3,98 (q, M-Teil vom ABM-Signal, $J_{BM}$=$J_{4,5}$=9,5 Hz, 1H, C(4)-H); 5,34 ( d, J=9,5 Hz, 1H, C(5)-H); 6,87 und 7,5 (je m, 4H, $C_6H_4F$); 7,0 (s, 5H, $C_6H_5$).

Beispiel 19

(±) 4(R*),5(R*),7(S*)-5-[1-Hydroxy-(m-fluorophenyl]-methyl-1-methyl-4-phenylpyrrolidin-2-on

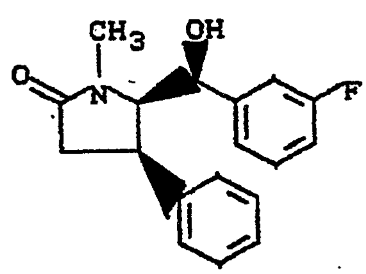

5,95 g (0,02 mol) der Titelverbindung aus Beispiel 17 wurden in 98 ml abs. Methanol gelöst, mit 0,77 g (0,02 mol) Natriumborhydrid versetzt und auf 60°C erhitzt bis im DC kein Ausgangsmaterial mehr vorhanden war (2-3 h). Man goß auf 200 ml Phosphatpufferlösung (pH=4) saugte ab und wusch gut mit Wasser nach. Man erhielt nach Trocknung im Hochvakuum 4,92 g (82,2% der Theorie) der Titelverbindung die direkt weiter umgesetzt wurden.

$^1$H-NMR (200 MHz, $CDCl_3$/DMSO): δ = 2,18 und 2,38 (ABM-Signal, $J_{AB}$=16 Hz, $J_{AM}$=9 Hz, $J_{BM}$=12,5 Hz, 2H, C(3)-H); 3,04 (s, 3H, N-$CH_3$); 3,82 (dt, M-Teil vom ABM-Signal, $J_{4,5}$=9 Hz, 1H, C(4)-H); (4,24 (dd, J=9 Hz, J=2 Hz, 1H, C(5)-H); 4,77 (dd, J=2 Hz, J=4 Hz, 1H, C(7)-H); 5,19 (d, J=4 Hz, 1H, OH); 6,37 (d, J=10 Hz, 1H, aus $C_6H_4F$); 6,55 (d, J=7,5 Hz, 1H, aus $C_6H_4F$); 6.75, 7,07 und 7,18 (je m, $C_6H_5$ und $C_6H_4F$).

Beispiel 20

(±) 4(R*),5(R*),7(S*)-5-[1-Hydroxy-1-m-chlorphenyl]-methyl-1-methyl-4-phenylpyrrolidin-2-on

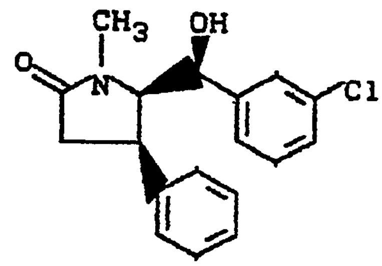

Man verfuhr analog Beispiel 19. Eingesetzt wurden 5,17 g (0,0164 mol) (±) 4(R*),5(R*)-5-[3-Chlor]benzolyl-1-methyl-4-phenylpyrrolidin-2-on (hergestellt analog Beispiel 7). Man erhielt 4,79 g (92,4% der Theorie) der Titelverbindung.
$^1$H-NMR (200 MHz, $CDCl_3$/DMSO): δ = 2,15 und 2,45 (A und B-Teil vom ABM-Signal, $J_{AB}$=16 Hz, $J_{AM}$=9 Hz, $J_{BM}$=12,5 Hz, 2H, C(3)-H); 3,0 (s, 3H, N-$CH_3$); 3,81 (dt, $J_{4,5}$=8 Hz, 1H, C(4)-H); 4,26 (dd, J=8 Hz, J=2,5 Hz, 1H, C(5)-H); 4,75 (dd, J=2,5 Hz, J=4,5 Hz, 1H, C(7)-H); 6,56 (s, 1H, C(2)-$C_6H_4Cl$); 6,70, 7,05 und 7,15 (je m, 8H, $C_6H_5$ und $C_6H_3Cl$).

Beispiel 21

(±) 4(R*),5(R*),7(S*)-5-(1-Hydroxy-1-p-chlorphenyl)-methyl-1-methyl-4-phenylpyrrolidin-2-on

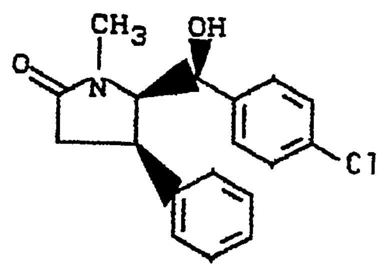

Man verfuhr analog Beispiel 19. Eingesetzt wurden 5,45 g (0,02 mol) der Titelverbindung aus Beispiel 16. Man erhielt 5,47 g (99% der Theorie) der Titelverbindung.
$^1$H-NMR (200 MHz, $CDCl_3$/DMSO): δ = 2,15 (d, J=10 Hz, 2H, C(3)-H); 3,06 (s, 3H, N-$CH_3$); 3,82 8q, breit, J=10 Hz, 1H, C(4)-H); 4,26 (dd, J=8 Hz, J=2,5 Hz, 1H, C(5)-H); 4,72 (dd, J=2,5 Hz, J=4,5 Hz, 1H, C(7)-H); 5,25 (d, J=4,5 Hz, 1H, OH); 6,64 (d, J=9 Hz, 2H, AB-Signal von $C_6H_4Cl$); 7,04 (d, J=9 Hz, 2H, AB-Signal von $C_6H_4Cl$); 7,06 und 7,20 (je m, 5 H, $C_6H_5$).

Beispiel 22

(±) 3(S*),4(R*),5(R*),7(S*)-3-Hydroxy-5-[1-hydroxy-1- (m-fluoro)phenyl]methyl-1-methyl-4-phenylpyrrolidin-2-on

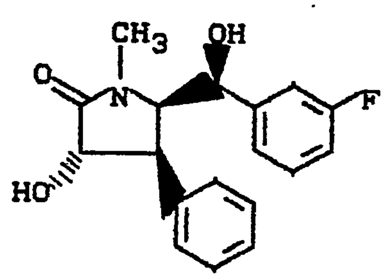

Man verfuhr analog Beispiel 9. Eingesetzt wurden 2 g (0,0067 mol) der Titelverbindung aus Beispiel 19. Man erhielt nach Chromatographie (s. Beispiel 19) 0,61 g (31% der Theorie) der reinen Titelverbindung. Retentionszeit: 6,07 min (HPLC, Hibar Fertigsäule 250-4, Lichrosorb Si 60 (5 µm), Eluens: Essigester/MeOH =20/1; 1 ml/min) 0,7 g (35% der Theorie) der Ausgangsverbindung konnten zurückgewonnen werden.

$^1$H-NMR-(200 MHz, $CDCl_3$/DMSO): δ = 3,16 (s, 3H, N-$CH_3$); 3,64 (dd, J=10,5 Hz, J=7,5 Hz, 1H, C(4)-H); 4,13 (dd, J=5,5 Hz, J=10,5 Hz, 1H, C(3)-H); 4,26 (dd, J=7,5 Hz, J=1 Hz, 1H, C(5)-H); 4,83 (dd, J=1 Hz, J=4 Hz, 1H, C(7)-H); 5,23 (d, J=5,5 Hz, 1H, C(3)-OH, austauschbar mit $D_2O$); 5,38 (d, J=4 hz, 1H, C(7)-OH, austauschbar mit $D_2O$); 6,40 (d, J=10 Hz, 1H, aus $C_6H_4F$); 6,58 (d, J=7,5 Hz, 1H aus $C_6H_4F$); 6,80 und 7,13 (je m, 7H, $C_6H_5$ und aus $C_6H_4F$).

$C_{18}H_{18}NO_3$ (315,3) Ber.: C 68,6 H 5,8 F 6,0

Gef.: C 68,6 H 5,8 F 5,9

Beispiel 23

(±) 3(S*),4(R*),5(R*),7(S*)-3-Hydroxy-5-[1-hydroxy-p-Chlorophenyl]methyl-1-methyl-4-phenylpyrrolidin-2-on

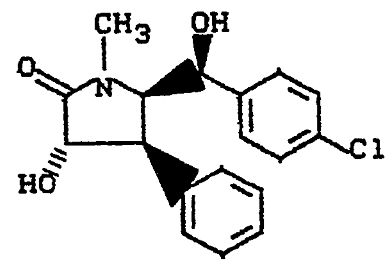

Man verfuhr analog Beispiel 9. Eingesetzt wurden 2,0 g (0,0063 mol) der Titelverbindung aus Beispiel 21. Man erhielt 0,71 g (31 %der Theorie) der reinen Titelverbindung mit Retentionszeit = 6, 34min (Bedingungen s. Beispiel 22) und Schmp.: 254-7°C. 0,7 g des Ausgangsmaterial konnten unverändert zurückgewonnen werden.

$^1$H-NMR (300 MHz, $CDCl_3$/DMSO): δ = 3,11 (s, 3H, N-$CH_3$); 3,58 (dd, J=7,5 Hz, J=10,5 Hz, 1H, C(4)-H); 3,90 (dd, J=10,5 Hz, J=5,5 Hz, 1H, C(3)-H); 4,20 (dd, J=7,5 Hz, J=2 Hz, 1H, C(5)-H); 4,50 (d, J=5,5 Hz, 1H, C(3)-OH, mit $D_2O$ austauschbar); 4,70 (t, J=2 Hz, 1H, C(7)-H); 4,80 (d, J=2 Hz, 1H, C(7)-OH, mit $D_2O$ austauschbar); 6,58 und 6,98 (AB-Signal, $J_{AB}$=8,3 Hz, 4H, $C_6H_4Cl$); 7,13 (m, 5H, $C_6H_5$).

**Patentansprüche**

1. Verfahren zur Herstellung von C(3)-C(4)-trans-konfigurierten γ-Butyrolactamen der allgemeinen Formel (I)

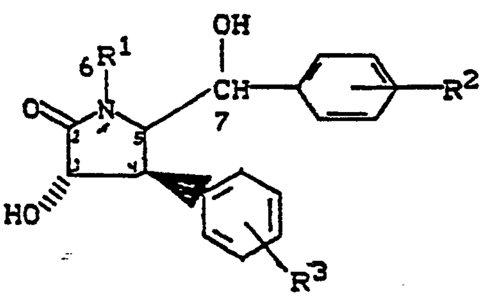

(I),

in welcher

$R^1$ für Wasserstoff, Alkyl, Aryl oder Aralkyl mit jeweils bis zu 10 Kohlenstoffatomen steht,

und

$R^2$ und R3 gleich oder verschieden sind und für Wasserstoff, Alkyl, Aryl, Aralkyl, Alkoxy, Aryloxy oder Aralkoxy mit jeweils bis zu 10 Kohlenstoffatomen, Acyl mit bis zu 18 Kohlenstoffatomen, Trifluormethyl, Trifluormethoxy, Nitro, Hydroxy, Halogen, Amino, Carboxy, Sulfo, Dialkylamino mit bis zu 4 Kohlenstoffatomen in den Alkylgruppen oder Acylamino mit bis zu 18 Kohlenstoffatomen steht,

mit der Ausnahme, daß $R^1$ nicht Methyl bedeutet, wenn $R^2$ und $R^3$ Wasserstoff bedeuten, in Form ihrer Isomeren, Isomerengemische, Racemate oder optischen Antipoden, dadurch gekennzeichnet, daß man Verbindungen der allgemeinen Formel (II)

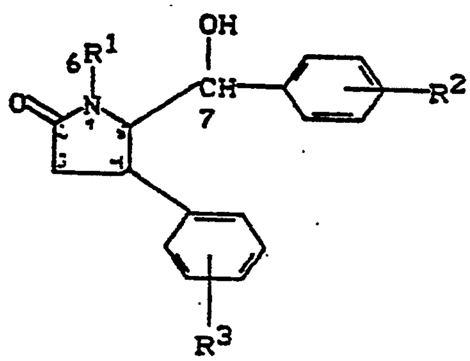

(II)

in welcher

$R^1$, $R^2$ und $R^3$ die oben angegebene Bedeutung haben, in inerten organischen Lösungsmitteln in Anwesenheit einer zur Enolatbildung geeigneten Base oder eines tertiären Amins gegebenenfalls in Anwesenheit eines zur Reduktion von Hydroperoxiden geeigneten Hilfsstoffs mit anorganischen oder organischen Peroxoverbindungen, Sauerstoff, Ozon oder Sauerstoffüberträgern bei Temperaturen zwischen −100°C und +20°C oxidiert.

2. Verfahren nach Anspruch 1, dadurch gekennzeichnet, daß
$R^1$ für Alkyl mit bis zu 4 Kohlenstoffatomen steht und
$R^2$ und $R^3$ gleich oder verschieden sind und für Alkyl mit bis zu 4 Kohlenstoffatomen, Alkoxy mit bis zu 4 Kohlenstoffatomen, Phenyl, Benzyl, Phenoxy, Benzyloxy, Benzoyl, Acetyl, Trifluormethyl, Trifluormethoxy, Nitro, Fluor, Chlor, Brom, Hydroxy, Amino, Dimethylamino, Diethylamino, Acetylamino, Carboxy oder Sulfo steht.

3. Verfahren nach Anspruch 1, dadurch gekennzeichnet, daß man als Hilfsstoffe Phosphite verwendet.

4. Verfahren nach Anspruch 1, dadurch gekennzeichnet, daß man die Oxidation mit Sauerstoff in Tetrahydrofuran oder Hexamethylphosphorsäuretriamid oder Gemischen dieser Lösungsmittel in Gegenwart von Triethylphosphit durchführt.

5. Verbindungen der allgemeinen Formel II

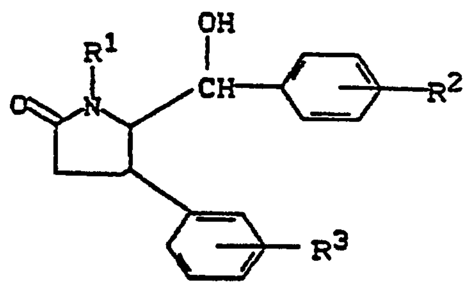

(II)

in welcher
$R^1$ für Wasserstoff, Alkyl, Aryl oder Aralkyl mit jeweils bis zu 10 Kohlenstoffatomen steht, und
$R^2$ und $R^3$ gleich oder verschieden sind und für Wasserstoff, Alkyl, Aryl, Aralkyl, Alkoxy, Aryloxy oder Aralkoxy mit jeweils bis zu 10 Kohlenstoffatomen, Acyl mit bis zu 18 Kohlenstoffatomen, Trifluormethyl, Trifluormethoxy, Nitro, Hydroxy, Halogen, Amino, Carboxy, Sulfo, Dialkylamino mit bis zu 4 Kohlenstoffatomen in den Alkylgruppen oder Acylamino mit bis zu 18 Kohlenstoffatomen steht, mit der Ausnahme, daß $R^1$ nicht Methyl bedeutet, wenn $R^2$ und $R^3$ Wasserstoff bedeuten, in Form ihrer Isomeren, Isomerengemische, Racemate oder optischen Antipoden.

## Claims

1. Process for the preparation of C(3)-C(4)-transconfigurated γ-butyrolactams of the general formula (I)

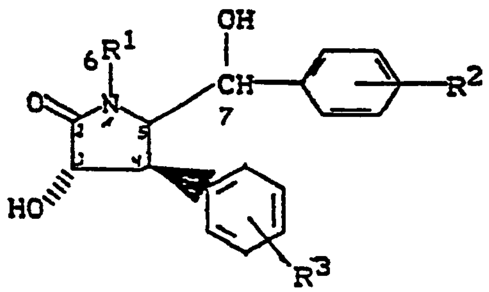

(I),

in which

$R^1$ represents hydrogen, alkyl, aryl or aralkyl with in each case up to 10 carbon atoms and

$R^2$ and $R^3$ are identical or different and represent hydrogen, alkyl, aryl, aralkyl, alkoxy, aryloxy or aralkoxy with in each case up to 10 carbon atoms, acyl with up to 18 carbon atoms, trifluoromethyl, trifluoromethoxy, nitro, hydroxyl, halogen, amino, carboxyl, sulpho, dialkylamino with up to 4 carbon atoms in the alkyl groups or acylamino with up to 18 carbon atoms, with the exception that $R^1$ does not denote methyl if $R^2$ and $R^3$ denote hydrogen, in the form of their isomers, isomer mixtures, racemates of optical antipodes, characterised in that compounds of the general formula (II)

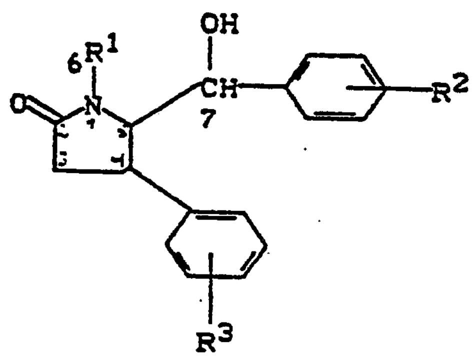

(II)

in which

$R^1$, $R^2$ and $R^3$ have the abovementioned meaning, are oxidised with inorganic or organic peroxo compounds, oxygen, ozone or oxygen transfer agents at temperatures between –100°C and +20°C, in inert organic solvents in the presence of a base, suitable for enolate formation or of a tertiary amine, if appropriate in the presence of an auxiliary suitable for reducing hydroperoxides.

2. Process according to Claim 1, characterised in that

$R^1$ represents alkyl with up to 4 carbon atoms and

$R^2$ and $R^3$ are identical or different and represent alkyl with up to 4 carbon atoms, alkoxy with up to 4 carbon atoms, phenyl, benzyl, phenoxy, benzyloxy, benzoyl, acetyl, trifluoromethyl, trifluoromethoxy, nitro, fluorine, chlorine, bromine, hydroxyl, amino, dimethylamino, diethylamino, acetylamino, carboxyl or sulpho.

3. Process according to Claim 1, characterised in that phosphites are used as the auxiliaries.

4. Process according to Claim 1, characterised in that the oxidation is carried out with oxygen in tetrahydrofuran or hexamethylphosphoric acid triamide or mixtures of these solvents in the presence of triethyl phosphite.

5. Compounds of the general formula II

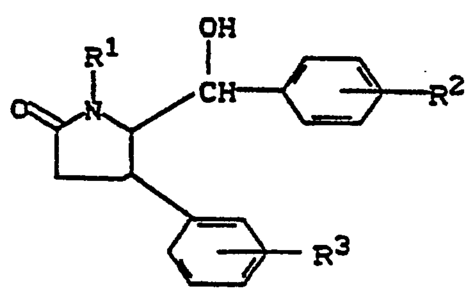

(II)

in which

$R^1$ represents hydrogen, alkyl, aryl or aralkyl with in each case up to 10 carbon atoms and
$R^2$ and $R^3$ are identical or different and represent hydrogen, alkyl, aryl, aralkyl, alkoxy, aryloxy or aralkoxy with in each case up to 10 carbon atoms, acyl with up to 18 carbon atoms, trifluoromethyl, trifluoromethoxy, nitro, hydroxyl, halogen, amino, carboxyl, sulpho, dialkylamino with up to 4 carbon atoms in the alkyl groups or acylamino with up to 18 carbon atoms, with the exception that $R^1$ does not denote methyl if $R^2$ and $R^3$ denote hydrogen, in the form of their isomers, isomer mixtures, racemates or optical antipodes.

**Revendications**

1. Procédé de préparation de γ-butyrolactames de configuration C(3)-C(4)-trans de formule générale (I):

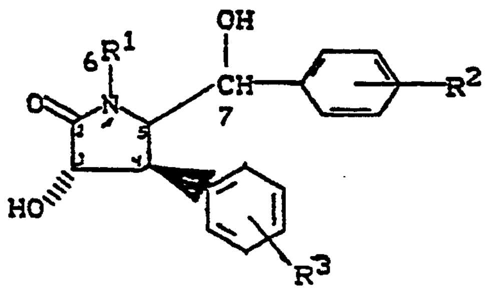

(I),

dans laquelle
$R^1$ représente un atome d'hydrogène, un groupe alkyle, un groupe aryle ou un groupe aralkyle contenant chacun jusqu'à 10 atomes de carbone, et
$R^2$ et $R^3$ sont identiques ou différents et représentent chacun un atome d'hydrogène, un groupe alkyle, un groupe aryle, un groupe aralkyle, un groupe alcoxy, un groupe aryloxy ou un groupe aralcoxy contenant chacun jusqu'à 10 atomes de carbone, un groupe acyle contenant jusqu'à 18 atomes de carbone, un groupe trifluorométhyle, un groupe trifluorométhoxy, un groupe nitro, un groupe hydroxyle, un atome d'halogène, un groupe amino, un groupe carboxyle, un groupe sulfo, un groupe dialkylamino contenant jusqu'à 4 atomes de carbone dans les groupes alkyle, ou un groupe acylamino contenant jusqu'à 18 atomes de carbone, avec cette exception que $R^1$ ne représente pas un groupe méthyle lorsque $R^2$ et $R^3$ représentent chacun un atome d'hydrogène, sous forme de leurs isomères, de leurs mélanges d'isomères, de leurs racémates ou de leurs antipodes optiques, caractérisé en ce qu'on oxyde des composés de formule générale (II):

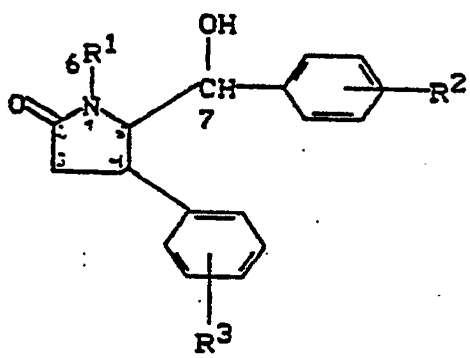

(II)

dans laquelle
$R^1$, $R^2$ et $R^3$ ont les significations indiquées ci-dessus, dans des solvants organiques inertes, en présence d'une base appropriée pour la formation d'énolate ou d'une amine tertiaire, éventuellement en présence d'une substance auxiliaire appropriée pour la réduction d'hydroperoxydes, avec des peroxo-composés inorganiques ou organiques, de l'oxygène, de l'ozone ou des agents de transfert d'oxygène, à des températures comprises entre −100°C et +20°C.

2. Procédé selon la revendication 1, caractérisé en ce que
$R^1$ représente un groupe alkyle contenant jusqu'à 4 atomes de carbone, et
$R^2$ et $R^3$ sont identiques ou différents et représentent chacun un groupe alkyle contenant jusqu'à 4 atomes de carbone, un groupe alcoxy contenant jusqu'à 4 atomes de carbone, un groupe phényle, un groupe benzyle, un groupe phénoxy, un groupe benzyloxy, un groupe benzoyle, un groupe acétyle, un groupe trifluorométhyle, un groupe trifluorométhoxy, un groupe nitro, un atome de fluor, un atome de chlore, un atome de brome, un groupe hydroxyle, un groupe amino, un groupe diméthylamino, un groupe diéthyl-

amino, un groupe acétylamino, un groupe carboxyle ou un groupe sulfo.

3. Procédé selon la revendication 1, caractérisé en ce que, comme substances auxiliaires, on utilise des phosphites.

4. Procédé selon la revendication 1, caractérisé en ce qu'on effectue l'oxydation avec de l'oxygène dans du tétrahydrofuranne ou du triamide d'acide hexaméthyl-phosphorique ou des mélanges de ces solvants en présence de phosphite de triéthyle.

5. Composés de formule générale (II):

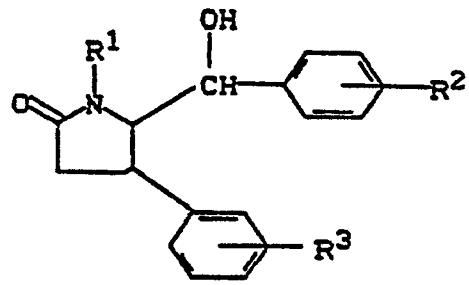

(II)

dans laquelle
$R^1$ représente un atome d'hydrogène, un groupe alkyle, un groupe aryle ou un groupe aralkyle contenant chacun jusqu'à 10 atomes de carbone, et
$R^2$ et $R^3$ sont identiques ou différents et représentent chacun un atome d'hydrogène, un groupe alkyle, un groupe aryle, un groupe aralkyle, un groupe alcoxy, un groupe aryloxy ou un groupe aralcoxy contenant chacun jusqu'à 10 atomes de carbone, un groupe acyle contenant jusqu'à 18 atomes de carbone, un groupe trifluorométhyle, un groupe trifluorométhoxy, un groupe nitro, un groupe hydroxyle, un atome d'halogène, un groupe amino, un groupe carboxyle, un groupe sulfo, un groupe dialkylamino contenant jusqu'à 4 atomes de carbone dans les groupes alkyle ou un groupe acylamino contenant jusqu'à 18 atomes de carbone, avec cette exception que $R^1$ ne représente pas un groupe méthyle lorsque $R^2$ et $R^3$ représentent chacun un atome d'hydrogène, sous forme de leurs isomères, de leurs mélanges d'isomères, de leurs racémates ou de leurs antipodes optiques.